# EUROPEAN PATENT APPLICATION

(11) **EP 0 834 319 A1**
(43) Date of publication of application: **08.04.1998**
(21) Application number: 96307312.7
(22) Date of filing: 07.10.1996
(51) Int. Cl.: A61K 33/34

(54) **Mineral and vitamin combinations in arthritic pain**

(71) Applicant: Lalvani, Kartar, Dr., London NW10 6SU (GB)
(72) Inventor: Lalvani, Kartar, Dr., London NW10 6SU (GB)

(57) **Abstract**

The invention relates to a novel combination of the nutritional elements, calcium, magnesium, zinc, copper, manganese and vitamin D for the management of osteoarthritic and rheumatoid arthritic pain in humans. This should improve the integrity and structure of subchondral and periarticular bone in arthritic joints.

## Description

This invention relates to a novel combination of nutritional elements which in combination have a therapeutic significance on the management of osteoarthritic and rheumatoid arthritic pain in humans.

Osteoarthritis is a complex disease which requires the broad skills of a multidisciplinary team for effective management.

Current drug based forms of therapy have not been very satisfactory and questions have been raised over the long term use of NSAIDS, (Non Steroidal Anti-inflammatory Drugs) due to various side-effects, particularly in the gastro-intestinal tract.

It is therefore the object of this invention to control the pain associated with Osteoarthritis and Rheumatoid arthritis without the side effects.

The use of this combination in the treatment of Rheumatoid Arthritis is also recommended.

In most types of arthritis subchondral bone is in a state of increased turnover and accelerated bone remodelling [1,2]. We propose that calcium supplementation improves the integrity and structure of subchondral and periarticular bone in arthritic joints, especially where erosions, microfractures and secondary localized osteoporosis are present. This could result in decreased inflammation in the affected joints with reduction in arthritic symptoms including pain.
[1] Erosion of articular cartilage in **osteoarthritis** exposes subchondral bone to increased weight-bearing stress and repetitive trauma which results in subchondral microfractures. Attempted repair of these microfractures leads to sclerotic changes and increased bone turnover and bone remodelling.
[2] The inflammatory pannus of **rheumatoid arthritis** leads to erosion and destruction of the articular cartilage and subjacent bone resulting in secondary localized periarticular osteoporosis.

The formula is to be composed of calcium with one or more of the minerals magnesium, zinc, copper, manganese and vitamin D.

Calcium is a major constituent part of bone and is commonly taken to help maintain strong bones. Zinc plays a role at the level of stimulation of DNA synthesis in bone cells. Vitamin D is involved in the formation of a carrier protein required for the absorbtion of calcium. Magnesium is also a constituent of bone and has been shown to help build peak bone density. Two thirds of the body's magnesium is stored in bones.

The addition of magnesium in the combination prevents calcium-oxalate stone formation.

The calcium may be present in an inorganic or organic form such as carbonate, citrate, lactate etc.

The zinc may be present as sulphate or in other organic and inorganic forms.

The vitamin D may be present as cholecalciferol or ergocalciferol.

The magnesium may be present as sulphate, citrate, oxide, hydroxide and other organic or inorganic forms.

Copper and manganese may be present as sulphate, chloride, gluconate or in any other organic or inorganic forms.

The following examples illustrate the invention and the advantages thereof. These examples are given by way of illustration only, and are not to be construed as limiting the invention in scope or in spirit, as many modifications will be apparent from this disclosure to those n this art.

The combination may consist of
Example 1
   Calcium¹ with magnesium², zinc³ copper⁵, manganese⁶ and vitamin D⁴.
Example 2
   Calcium¹ with magnesium², zinc³, copper⁵ and vitamin D⁴.
Example 3
   Calcium¹ with magnesium², zinc³, manganese⁶ and vitamin D⁴.
Example 4
   Calcium¹ with magnesium², and vitamin D⁴.
Example 5
   Calcium¹ with zinc³ and vitamin D⁴.
Example 6
   Calcium¹ with magnesium² and zinc³.
Example 7
   Calcium¹ with magnesium².
Example 8
   Calcium¹ with zinc³.
Example 9
   Calcium¹ with vitamin D⁴.

¹The daily dosage of calcium may range between : 100-5000mg
²The daily dosage of magnesium may range between: 50-2500mg
³The daily dosage of zinc may range between : 2-100mg
⁵The daily dosage of copper may range between : 0.1mg-6mg
⁶The daily dosage of manganese may range between: 0.5mg-20mg
⁴The daily dosage of vitamin D may range between: 25-2000IU

The invention covers the ingredients in tablet or capsule a liquid or injectable form.

The above combinations, may or may not be combined with herbal materials.

## Claims

1. A product for the management of osteoarthritic and rheumatoid arthritics pain in humans, comprising calcium with one or more of the nutritional elements, magnesium, zinc, vitamin D, copper and manganese, in the form of a tablet, capsule, liquid and injection.

2. A product as claimed in claim 1 wherein the calcium concentration is 100-5000mg.

3. A product as claimed in claim 1 with 100-5000mg calcium, 50-2500mg magnesium, and 25-2000iu vitamin D.

4. A product as claimed in claim 1 with 100-5000mg calcium, 50-2500mg magnesium, 2-100mg zinc, 0.1-6mg copper, and 25-2000iu vitamin D.

5. A product as claimed in claim 1 with 100-5000mg calcium, 50-2500mg magnesium, 2-100mg zinc, 0.5mg-20mg manganese, and 25-2000iu vitamin D.

6. A product as claimed in claim 1 comprising 100-5000mg calcium with 50-2500mg magnesium, 2-100mg zinc, 0.1mg-6mg copper, 0.5mg-20mg manganese and 25-2000iu vitamin D.

7. A product as claimed in claim 1 with 100-5000mg calcium, 2-100mg zinc and 25-2000iu vitamin D.

8. A product as claimed in claim 1 with 100-5000mg calcium, 50-2500mg magnesium and 2-100mg zinc.

9. A product as claimed in claim 1 with 100-5000mg calcium and 50-2500mg magnesium.

10. A product as claimed in claim 1 with 100-5000mg calcium and 2-100mg zinc.

11. A product as claimed in claim 1 with 100-5000mg calcium and 25-2000iu vitamin D.

12. The dosage limits for the product described (in claims 2. to 11.) represent amounts for daily intake.
